# EUROPEAN PATENT SPECIFICATION

(11) **EP 1 299 100 B1**
(45) Date of publication and mention of the grant of the patent: **25.07.2007**
(21) Application number: 01942248.4
(22) Date of filing: 12.06.2001
(51) Int. Cl.: A61K 31/40, A61K 31/445, A61P 17/00

(54) **METHOD OF TREATING HOT FLASHES, USING TACHYKININ RECEPTOR ANTAGONIST**
METHODE ZUR BEHANDLUNG VON HITZEWALLUNGEN, DURCH VERWENDUNG EINES TACHIKININ-REZEPTOR ANTAGONISTEN
METHODE DE TRAITEMENT DE BOUFFEES DE CHALEUR, AU MOYEN D'UN ANTAGONISTE DU RECEPTEUR DE LA TACHYKININE

(30) Priority: 12.06.2000 US 211116 P
(43) Date of publication of application: 09.04.2003
(73) Proprietor: THE UNIVERSITY OF ROCHESTER, Rochester, NY 14627-0140 (US)
(72) Inventor: GUTTUSO, Thomas, J., Jr., Rochester, NY 14610 (US)
(74) Representative: Miles, John Stephen
(86) International application number: PCT/US2001/040924
(87) International publication number: WO 2001/095904

(56) References cited:
- WO-A1-96/24353
- WO-A1-98/43639
- WO-A1-99/09987
- FISHER L ET AL: "Tachykinin receptors mediating contractions of oestrogen-primed rat uterus: Classification using non-peptide antagonists" CLINICAL AND EXPERIMENTAL PHARMACOLOGY AND PHYSIOLOGY, vol. 26, no. 9, September 1999 (1999-09), pages 729-735, XP002306101 ISSN: 0305-1870
- MOODLEY NALLINI ET AL: "NK2 receptors mediate tachykinin-induced contractions of rat uterus during the oestrous cycle" EUROPEAN JOURNAL OF PHARMACOLOGY, vol. 376, no. 1-2, 2 July 1999 (1999-07-02), pages 53-60, XP002306102 ISSN: 0014-2999

## Description

This application claims priority benefit of U.S. Provisional Patent Application Serial No. 60/211,116, filed June 12, 2000.

### FIELD OF THE INVENTION

The present invention relates generally to the preparation of medicaments for treating symptoms of hormonal variation, including hot flashes.

### BACKGROUND OF THE INVENTION

Hot flashes or flushing occur commonly in menopausal women. This is characterized by a sudden onset of warmth in the face and neck and often progressing to the chest. Such an episode generally lasts several minutes and is evidenced by a visible flushing of the skin. Often such episodes are accompanied by sweating, dizziness, nausea, palpitations and diaphoresis. Such symptoms can disrupt sleep and interfere with the quality of life. Although the cause of hot flashes are not completely understood, they are thought to be a disorder of thermoregulation resulting from a transient lowering of the hypothalamic temperature regulatory set point (Kronenberg et al., "Thermoregulatory Physiology of Menopausal Hot Flashes: A Review," Can. J. Physiol. Pharmacol., 65:1312-1324 (1987)). In post-menopausal woman, the cause of such hot flashes is believed to be a consequence of declining estrogen levels. Thus, it is not surprising that hot flashes also occur in a high percentage of women taking the anti-estrogen drug tamoxifen.

Men may also have hot flashes following androgen-deprivation therapy (from bilateral orchiectomy or treatment with a gonadotrophin-releasing-hormone agonist) for metastatic prostate cancer.

Although estrogen replacement therapy is the most direct and effective treatment for hot flashes in women, there are women for whom such therapy is contraindicated, i.e., women with breast cancer or a strong family history of breast cancer, a history of clotting, severe migraine, or who are averse to taking the drug.

In these women, there are alternative medications to prevent or treat the serious consequences of menopause, such as osteoporosis and raised serum lipid levels. Included in this category are the selective estrogen-receptor modulators (SERMs), such as raloxifene (see U.S. Patent No. 5,534,526 to Cullinan), which selectively bind to and activate the estrogen receptors of some tissues such as bone, and block the receptors of others, i.e., breast and uterus. In so doing, they lack the negative impact that prolonged estrogen therapy may have on these organs. However, in contrast to estrogen, SERMs are not as effective in preventing hot flashes.

Other than estrogen-replacement therapy, there are no effective means to alleviate hot flashes. Low dose oral megestrol acetate, a progestational agent, was shown to reduce the frequency of hot flashes in both men and women in a short term study (Loprinzi et al., "Megestrol Acetate for the Prevention of Hot Flashes," N. Engl. J. Med. 331:347-351 (1994)). However, chronic adrenal insufficiency can be a side effect of low dose megestrol acetate when taken long term. Transdermal clonidine, a centrally active α-agonist, had only a moderate effect on the frequency and severity of hot flashes in tamoxifen-treated women (Goldberg et al., "Transdermal Clonidine for Ameliorating Tamoxifen-induced Hot Flashes," J. Clin. Onc. 12:155-158 (1994)).

Accordingly, there is a need for an alternative method of treating symptoms of hormonal variation, including hot flashes, which overcomes the deficiencies in the relevant art

### SUMMARY OF THE INVENTION

A first aspect of the present invention relates to the use of a tachykinin receptor antagonist in the preparation of a medicament for treating hot flashes in a patient.

A second aspect of the present invention relates to the use of a tachykinin receptor antagonist in the preparation of a medicament for treating a symptom of hormonal variation in a postmenopausal female patient.

The present invention provides an improved treatment for symptoms of hormonal variation, including hot flashes, which can be significantly uncomfortable and seriously affect one's quality of life. Tachykinin receptor antagonists, which can inhibit the activity of neurokinins on their receptors, can be administered in a manner which is effective to reduce or substantially eliminate the occurrence or severity of hot flashes. Current trials involving the administration of various tachykinin receptor antagonists for other uses have demonstrated that a number of such antagonists are well tolerated by patients.

### DETAILED DESCRIPTION OF THE INVENTION

A first aspect of the present invention relates to the use of a tachykinin receptor antagonist in the preparation of a medicament for treating hot flashes in a patient.

A second aspect of the present invention relates to the use of a tachykinin receptor antagonist in the preparation of a medicament for treating a symptom of hormonal variation in a patient.

Tachykinins are small peptides found in the central and peripheral nervous systems. Three different tachykinins have been identified in mammals: substance P, neurokinin A, and neurokinin B. Each of these acts as a neurotransmitter and neuromodulator (Maggi et al., "Tachykinin and Tachykinin Receptors," J. Auton. Pharmacol. 13:23-93 (1993); Nakanishi, "Mammalian Tachykinin Receptors," Ann. Rev. Neurosci. 14:123-136 (1991)). The diverse effects of these tachykinins are mediated by three receptors: NK₁, NK₂, and NK₃, all of which belong to the superfamily of G-protein coupled receptors (Maggi et al., "Neuropeptides as Regulators of Airway Function: Vasoactive Intestinal Peptide and the Tachykinins," Physiol. Rev. 151:277-322 (1995); Maggi, "The Mammalian Tachykinin Receptors," Gen. Pharmacol. 26:911-944 (1995)). The NK₁ receptor prefers substance P, the NK₂ receptor prefers neurokinin A, and the NK₃ receptor prefers neurokinin B.

In both the postmenopausal woman and the ovariectomized rat, there is a dramatic increase in neurokinin B gene expression in the hypothalamic arcuate nucleus (ArN)-in rat and human there is roughly a 2-fold and 15-fold increase, respectively, in ArN cells expressing-NKB, while in both rat and human there is a 2-fold increase in ArN NKB. grain density (Rance and Bruce, "Neurokinin B gene expression is increased in the arcuate nucleus of ovariectomized rats," Neuroendocrinology 60:337-345 (1994); Rance and Young, "Hypertrophy and increased gene expression of neurons containing neurokinin-B and substance-P messenger n'bonucleic acids in the hypothalami of postmenopausal women," - Endocrinol. 128:2239-2247 (1991)). In humans and rats, the enhanced NKB gene expression is isolated to the ArN. Furthermore, treatment of the ovariectomized rat or primate with estrogen completely prevents these ArN changes (Rance and Bruce, "Neurokinin B gene expression is increased in the arcuate nucleus of ovariectomized rats," Neuroendocrinology 60:337-345 (1994); Abel et al., "The effects of hormone replacement therapy on hypothalamic neuropeptide gene expression in a primate model of menopause," J. Clin. Endocrinol. & Metab. 84:2111-2118 (1999)). Since estrogen therapy in postmenopausal women is the most effective treatment of hot flashes, estrogen's effects on NKB expression in the ArN may be involved with estrogen's mechanism of action. This is supported by the observation that systemic injection of substance-P, a closely related tachykinin to NKB, results in a clinical hot flash in humans (Schaffalitzky De Muckadell et aL, "Flushing and plasma substance P concentration during infusion of synthetic substance P in normal man," Scand. J. Gastroenterology 21:498-502 (1986)).

It has been postulated that hot flashes result from a transient lowering of the hypothalamic temperature regulatory set point (Kronenberg and Downey, "Thermoregulatory physiology of menopausal hot flashes: a review," Canad. J. Physiol. Pharmacol. 65:1312-1324 (1987)). This results in a sudden perception of heat and activation of physiological cooling processes such as sweating and cutaneous vasodilation-i.e., a hot flash. The medial preoptic area (MPOA) is the principle nucleus regulating heat-loss physiology (Simerly and Swanson, "Projections of the medial preoptic nucleus: a Phaseolus vulgaris leucoagglutinin anterograde tract-tracing study in the rat," J. Comp. Neurology 270:209-242 (1988)).

Direct stimulation of the MPOA results in a transient physiological response that mimics a hot flash-cutaneous vasodilation, sweating, and panting (Day et al., "Thermoregulatory effects of preoptic area injections of noradrenaline in restrained and unrestrained rats," Brain Res. 174:175-179 (1979); Casper and Yen, "Neuroendocrinology of menopausal flushes: an hypothesis of flush mechanism," Clin. Endocrinol. 22:293-312 (1985)). The ArN has a major projection to the MPOA (Akesson et al., "Estrogen-concentrating hypothalamic and limbic neurons project to the medial preoptic nucleus," Brain Res. 451:381-385 (1988)).

The MPOA also receives its largest substance-P input from the ventromedial hypothalamus (VMH) (Yamano et al., "A substance P-containing pathway from the hypothalamic ventromedial nucleus to the medial preoptic area of the rat: an immunohistochemical analysis," Neuroscience 18:395-402 (1986)).

Without being bound by theory, it is believed that postmenopausal (as well as surgically or chemically induced) hot flashes result from overstimulation of the MPOA by tachykinin projections from the ArN and the VMH which are disinhibited by the low estrogen state. The overactivity of the ArN tachykinin cells is mediated by an upregulation of their α₂δ subunits of voltage-gated calcium channels which, in turn, increases membrane calcium permeability causing increased cellular activity and neurotransmitter production. This MPOA overstimulation eventually reaches a threshold when the MPOA activates a lowering of the thermoregulatory set point and, consequently, a hot flash is experienced

As used in the present invention, the tachykinin receptor antagonist can be an NK₁ receptor antagonist, an NK₂ receptor antagonist, an NK₃ receptor antagonist, or a tarhykinin receptor antagonist which has antagonist effects at more than one of the several NK receptors. Preferably, the tachykinin receptor antagonist is an NK₁ receptor antagonist, an NK₃ receptor antagonist, or an antagonist of both the NK₁ receptor and the NK₃ receptor.

In addition, various combinations of NK₁ receptor antagonists, NK₂ receptor antagonists, and NK₃ receptor antagonists can be administered together. For example, such combinations may include an NK₁ receptor antagonist in combination with an NK₂ receptor antagonist, an NK₁ receptor antagonist in combination with an NK₃ receptor antagonist, an NK₂ receptor antagonist in combination with NK₃ receptor antagonist, or an NK₁ receptor antagonist in combination with both an NK₂ receptor antagonist and an NK₃ receptor antagonist. One preferred combination is an NK₁ receptor antagonist together with an NK₃ receptor antagonist.

A number of NK receptor antagonists have been described in the art and can be prepared according to known procedures as identified, for example, in the following references: U.S. Patent No. 5,344,830 to Mills et al., U.S. Patent No. 5,554,627 to Lewis et al., U.S. Patent No. 5,554,641 to Horwell et al., U.S. Patent No. 5,563,161 to Huscroft et al., U.S. Patent No. 5,594,022 to Horwell et al., U.S. Patent No. 5,607,936 to Chiang et al., U.S. Patent No. 5,610,145 to Horwell et al., U.S. Patent No. 5,610,165 to MacCoss et al., U.S. Patent No. 5,612,336 to Lewis et al., U.S. Patent No. 5,624,947 to Keown et al., U.S. Patent No. 5,627,211 to Teall et al., U.S. Patent No. 5,633,266 to Baker et al., U.S. Patent No. 5,635,509 to Jacobs et al., U.S. Patent No. 5,633,281 to Teall et al., U.S. Patent No. 5,654,316 to Carruthers et al., U.S. Patent No. 5,663,352 to MacLeod et al., U.S. Patent No. 5,665,883 to Baker et al., U.S. Patent No. 5,688,960 to Shankar, U.S. Patent No. 5,696,123 to Dollinger et al., U.S. Patent No. 5,696,267 to Reichard et al., U.S. Patent No. 5,698,710 to Sisto et al., U.S. Patent No. 5,708,006 to Dollinger et al., U.S. Patent No. 5,719,156 to Shue et al., U.S. Patent No. 5,731,309 to Bernstein et al., U.S. Patent No. 5,760,018 to Baker et al., U.S. Patent No. 5,760,248 to Sisto et al., U.S. Patent No. 5,846,965 to MacKenzie et al., U.S. Patent No. 5,849,795 to Sisto et al., U.S. Patent No. 5,892,039 to Shue et al., U.S. Patent No. 5,919,803 to Giblin et al., U.S. Patent No. 5,922,744 to Harrison et al., U.S. Patent No. 5,935,972 to Naylor et al., U.S. Patent No. 5,945,428 to Shih et al., U.S. Patent No. 5,962,485 to Owens, U.S. Patent No. 5,968,923 to MacKenzie et al., U.S. Patent No. 5,968,929 to Blythin et al., U.S. Patent No. 6,013,652 to MacCoss et al., U.S. Patent No. 6,020,346 to Armour et al., U.S. Patent No. 6,046,195 to Haworth et al., U.S. Patent No. 6,060,469 to Baker et al., U.S. Patent No. 6,063,926 to Reichard et al., U.S. Patent No. 6,103,719 to Esser et al., U.S. Patent No. 6,110,919 to Howard et al., U.S. Patent No. 6,150,325 to Arcamone et al., U.S. Patent No. 6,204,265 to Reichard et al., U.S. Patent No. 6,207,678 to Monaghan et al., U.S. Patent No. 6,242,438 to MacKenzie et al., and U.S. Patent No. 6,235,732 to Dollinger et al., Wallace et al., "A double ring closing metathesis reaction in the rapid, enantioselective synthesis of NK-1 receptor antagonists," Org. Lett. 3(5):671-674 (2001), Reichard et al., "The design and synthesis of novel NK1/NK2 dual antagonists," Bioorg. Med. Chem. Lett. 10(20):2329-2332 (2000), Liu et al., "Synthesis of a substance P antagonist with a somatostatin scaffold: factors affecting agonism/antagonism at GPCRs and the role of pseudosymmetry," J. Med. Chem. 43(21):3827-3831 (2000), Nishi et al., "Combined tachykinin receptor antagonist: synthesis and stereochemical structure-activity relationships of novel morpholine analogues," Bioorg.Med. Chem. Lett. 10(15):1665-668 (2000), Rosen et al., "Synthesis and structure-activity relationships of CP-122,721, a second-generation NK-1 receptor antagonist," Bioorg. Med. Chem. Lett. 8(3):281-284 (1998), Caliendo et al., "Synthesis and in vitro activities of NK-1 antagonists derived from L-tryptophan," Farmaco 52(10):589-593 (1997), Kubota et al., "Spiro-substituted piperidines as neurokinin receptor antagonists: Design and synthesis of (+/-)-N-[2-(3,4-dichlorophenyl)-4-(spiro[isobenzofuran-1(3H),4'piperidin]-1'-yl)butyl]-N-methylbenzamide, YM-35375, as a new lead compound for novel neurokinin receptor antagonists," Chem. Pharm. Bull. (Tokyo) 46(2):351-354 (1998), Hirschmann et al., "Synthesis of potent cyclic hexapeptide NK-1 antagonists. Use of a minilibrary in transforming a peptidal somatostatin receptor ligand into an NK-1 receptor ligand via a polyvalent peptidomimetic," J. Med. Chem. 39(13):2441-2448 (1996), Caliendo et al., "Synthesis and in vitro activities of highly potent and selective tripeptide antagonists of the neurokinin NK-1 receptor," Farmaco 50(11):755-759 (1995), Karagiannis et al., "Synthesis of a potent antagonist of substance P by replacing the CH2SCH3 and the alpha-carboxamide groups of the methionine at [Orn6]-SP6-11 by benzyl ester groups," Int. J. Pept. Protein Res. 42(6):565-569 (1993), Rosen et al., "Synthesis, in vitro binding profile, and autoradiographic analysis of [3H]-cis-3-[(2-methoxybenzyl)amino]-2-phenylpiperidine, a highly potent and selective nonpeptide substance P receptor antagonist radioligand," J. Med. Chem. 36(21):3197-3201 (1993), Caliendo et al., "Synthesis and neurokinin antagonist activity of 2-benzylidene- and 2-benzyl-3-benzylamino quinuclidines," Farmaco 48(10):1359-1378 (1993), Manolopoulou et al., "Synthesis of potent antagonists of substance P by modifying the methionyl and glutaminyl residues of its C-terminal hexapeptide and without using D-amino acids," Int J. Pept. Protein Res. 41(4):411-414 (1993), Lawrence et aL, "Synthesis and substance P antagonist activity of naphthimidazolium derivatives," J. Med. Chem. 35(7):1273-1279 (1992), Aitken et al., "Synthesis, modeling and NK1 antagonist evaluation of a non-rigid cyclopropane-containing analogue of CP-99,994," Bioorg. Med. Chem. Lett 11(5):659-661 (2001), Reichard et al., "The design and synthesis of novel NK1/NK2 dual antagonists," Bioorg. Med. Chem. Lett. 10(20):2329-2332 (2000), Elling et al.,"Disulfide bridge engineering in the tachykinin NK1 receptor," Biochemistry 39(4):667-675 (2000). Other NK receptor antagonists, now known or hereafter developed, can also be used according to the present invention.

A number of suitable NK₁ receptor antagonists are currently in clinical trials for other indications. These include, without limitation: GR 203040 which is available from GlaxoSmithKline (Research Triangle Park, NC); CP 99994 which is available from Pfizer (Groton, CT); CP 122721 (Rosen et al., "Synthesis and structure-activity relationships of CP-122,721, a second-generation NK-1 receptor antagonist," Bioorg. Med. Chem. Lett. 8(3):281-284 (1998)) which is available from Pfizer, GR 205171 1 which is available from GlaxoSmithKline; PD 154075 which is available from Pfizer (Parke-Davis); FK 888 which is available from Fujisawa Healthcare, Inc. (Deerfield, IL); RP 67580 which is available from Aventis (Strasbourg, France); L 760735 which is available from Merck (Whitehouse Station, NJ); TAK 637 which is available from Takeda Pharmaceuticals N.A. (Lincolnshire, IL); R116301 which is available from Cinalfa AG (Läufelfingen, Switzerland); RPR 100893 or dapitant (CAS 153438-49-4), which is available from Aventis; L 754030 and its prodrug L 758298, both available from Merck; MK 869 which is available from Merck; SR 140333, which is available from Sanofi-Synthelabo (Malvem, PA); NKP 608, which is available from Novartis (East Hanover, NJ); GR 73632 which is available from GlaxoSmithKline; MEN 11467 which is available from Menarini Group (Firenze, Italy), as well as pharmaceutically acceptable salts thereof. Suitable salts can be prepared according to known techniques. Combinations of one or more NK₁ receptor antagonists can also be administered.

A number of suitable NK₂ receptor antagonists are currently in clinical trials for other indications. These include, without limitation: SR 48968 or saredutant (CAS No. 142001-63-6), which is available from Sanofi-Synthelabo; and MEN 10627 (Quartara et al., "A review of the design, synthesis and biological activity of the bicyclic hexapeptide tachykinin NK2 antagonist MEN 10627," Regul. Pept. 65(1):55-59 (1996); Caciagli et al., "Large-scale production of peptides using the solid-phase continuous flow method. Preparative synthesis of the novel tachykinin antagonist MEN 10627, "J. Pept. Sci. 3(3):224-230 (1997), which is available from Menarini Group, as well as pharmaceutically acceptable salts thereof. Suitable salts can be prepared according to known techniques. Combinations of one or more NK₂ receptor antagonists can also be administered.

A number of suitable NK₃ receptor antagonists are currently in clinical trials for other indications. These include, without limitation: SB-223412-A or talnetant hydrochloride (CAS 204519-66-4) which is available from GlaxoSmithKline, and SR 142801 or osanetant (CAS 160492-56-8) which is available from Sanofi-Synthelabo, as well as pharmaceutically acceptable salts thereof Suitable salts can be prepared according to known techniques. Combinations of one or more NK₃ receptor antagonists can also be administered.

The present invention requires administration of the tachykinin receptor antagonist under conditions effective to treat either a symptom of hormonal variation or, more specifically, hot flashes (whether hormonally, surgically, drug, or otherwise induced). The effective conditions typically involve administering an amount of such compound that is effective for the desired treatment. By treating the symptom of hormonal variation, including hot flashes, the present invention encompasses either reducing the number of symptomatic events, reducing the severity of symptomatic events, or both.

Effective amounts of the tachykinin receptor antagonist will depend upon the mode of administration, frequency of administration, and the type of pharmaceutical composition used to deliver the compound into a patient Generally, effective amounts of such compounds will be about 0.01 to about 300 mg/kg·body wt. per day, preferably about 0.1 to about 200 mg/kg·body wt. per day, more preferably about 1 to about 100 mg/kg body wt. per day. Typical daily doses will be from about 10 to about 5000 mg per day for an average adult patient of normal weight. While individual needs vary, determination of optimal ranges of effective amounts of each compound is within the skill of the art. For tachykinin receptor antagonists which are involved in clinical trials for other indications, the safe and effective dosages identified in such trials can be considered when selecting dosages for treatments according to the present invention.

The tachykinin receptor antagonists used according to the present invention can be administered alone or as a pharmaceutical composition, which includes the compound(s) and a pharmaceutically-acceptable carrier. In forms available from the above-listed manufacturers, the tachykinin receptor antagonists are typically provided as a pharmaceutical composition.

The pharmaceutical composition can also include suitable excipients, or stabilizers, and can be in solid or liquid form such as, tablets, capsules, powders, solutions, suspensions, or emulsions. Typically, the composition will contain from about 0.01 to 99 percent, preferably from about 5 to 95 percent of active compound(s), together with the carrier.

The tachykinin receptor antagonist, when combined with pharmaceutically or physiologically acceptable carriers, excipients, or stabilizers, whether in solid or liquid form such as, tablets, capsules, powders, solutions, suspensions, or emulsions, can be administered orally, parenterally, subcutaneously, intravenously, intramuscularly, intraperitoneally, by intranasal instillation, by implantation, by intracavitary or intravesical instillation, intraocularly, intraarterially, intralesionally, transdermally, or by application to mucous membranes, such as, that of the nose, throat, and bronchial tubes (i.e., inhalation).

For most therapeutic purposes, the tachykinin receptor antagonist can be administered orally as a solid or as a solution or suspension in liquid form, via injection as a solution or suspension in liquid form, or via inhalation of a nebulized solution or suspension.

The solid unit dosage forms can be of the conventional type. The solid form can be a capsule, such as an ordinary gelatin type containing the compounds of the present invention and a carrier, for example, lubricants and inert fillers such as, lactose, sucrose, or cornstarch. In another embodiment, these compounds are tableted with conventional tablet bases such as lactose, sucrose, or cornstarch in combination with binders like acacia, cornstarch, or gelatin, disintegrating agents, such as cornstarch, potato starch, or alginic acid, and a lubricant, like stearic acid or magnesium stearate.

For injectable dosages, solutions or suspensions of these materials can be prepared in a physiologically acceptable diluent with a pharmaceutical carrier. Such carriers include sterile liquids, such as water and oils, with or without the addition of a surfactant and other pharmaceutically and physiologically acceptable carrier, including adjuvants, excipients or stabilizers. Illustrative oils are those of petroleum, animal, vegetable, or synthetic origin, for example, peanut oil, soybean oil, or mineral oil. In general, water, saline, aqueous dextrose and related sugar solution, and glycols, such as propylene glycol or polyethylene glycol, are preferred liquid carriers, particularly for injectable solutions.

For use as aerosols, the compound in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

For transdermal routes, the compound is present in a carrier which forms a composition in the form of a cream, lotion, solution, and/or emulsion. The composition can be included in a transdermal patch of the matrix or reservoir type as are conventional in the art for this purpose.

It is also contemplated that administration of the tachykinin receptor antagonist can be carried out in combination with other suitable therapeutic treatments which are useful for treating symptoms of hormonal variation, including hot flashes.

The patient to be treated is any mammalian patient, preferably a human patient. The patient can be either a female patient or a male patient, although the ultimate cause of hot flashes can, of course, be markedly different for both groups of patients. For example, in female patients the hot flash is a primary symptom resulting from menopausal or postmenopausal hormonal variation. However, the hot flash can also be drug-induced by anti-estrogen compounds (e.g., tamoxifen, leuprolide acetate, etc.) or surgically-induced by removal of estrogen-producing tissues (e.g., total abdominal hysterectomy, bilateral salpingo-oophorectomy, etc.). In male patients, the hot flashes typically occur as a side-effect of androgen-dependent therapy for metastatic prostate cancer. They can be either surgically-induced (e.g., bilateral orchiectomy) or drug-induced (e.g., treatment with a gonadotrophin-releasing-hormone agonist, leuprolide acetate, etc.).

### EXAMPLES

The following examples are provided to illustrate embodiments of the present invention but are by no means intended to limit its scope.

### Example 1 - Effect of NK₃ Receptor Antagonist on Controlling Hot Flashes in Postmenopausal Women

Postmenopausal women who report seven (7) or more hot flashes a day will participate in the study. For each patient, serum follicle stimulating hormone and estradiol levels will need to be in the postmenopausal range.

Talnetant hydrochloride (GlaxoSmithKline) is to be administered as an NK₃ receptor antagonist. Patients will be randomly assigned to talnetant hydrochloride treatment or placebo treatment using a double-blind protocol. Dosing of the talnetant hydrochloride will correspond to that which is found to be safe and appropriate in phase 1-3 clinical trials for other demonstrated uses. Low, medium, and high therapeutic dosing of talnetant hydrochloride will be compared against placebo treatment.

To monitor efficacy of placebo and medication, patients will record the frequency and severity of hot flashes in a daily diary. Data from the hot flash daily diaries will be analyzed by ANOVA for statistical significance, considering the talnetant hydrochloride dosage.

### Example 2 - Effect of NK₁ Receptor Antagonist on Controlling Hot Flashes in Postmenopausal Women

Postmenopausal women who report seven (7) or more hot flashes a day will participate in the study. For each patient, serum follicle stimulating hormone and estradiol levels will need to be in the postmenopausal range.

Dapitant (Aventis) is to be administered as an NK₁ receptor antagonist. Patients will be randomly assigned to dapitant treatment or placebo treatment using a double-blind protocol. Dosing of the dapitant will correspond to that which is found to be safe and appropriate in phase 1-3 clinical trials for other demonstrated uses. Low, medium, and high therapeutic dosing of dapitant will be compared against placebo treatment.

To monitor efficacy of placebo and medication, patients will record the frequency and severity of hot flashes in a daily diary. Data from the hot flash daily diaries will be analyzed by ANOVA for statistical significance, considering the dapitant dosage.

## Claims

1. Use of a tachykinin receptor antagonist in the preparation of a medicament for treating hot flashes in a patient.

2. The use according to claim 1 or 19, wherein the tachykinin receptor antagonist is an NK₁ receptor antagonist, an NK₂ receptor antagonist or an NK₃ receptor antagonist, or a combination thereof.

3. The use according to claim 2, wherein the NK₁ receptor antagonist is selected from the group consisting of GR 203040, CP 99994, CP 122721, GR 205171, PD 154075, FK 888, RP 67580, L 76035, TAK 637, R 116301, dapitant, L 754030, L 758298, MK 869, SR 140333, NKP 608, GR 73632, MEN 11467, and combinations thereof.

4. The use according to claim 2, wherein the NK₂ receptor antagonist is selected from the group consisting of saredutant, MEN 10627, and combinations thereof.

5. The use according to claim 2, wherein the NK₃ receptor antagonist is selected from the group consisting of talnetant hydrochloride, osanetant, and combinations thereof.

6. The use according to claim 1, wherein the medicament comprises the tachykinin receptor antagonist in an amount of about 10 to about 5000 mg per daily dose.

7. The use according to claim 1, wherein the patient is a female patient.

8. The use according to claim 7, wherein the female patient is postmenopausal.

9. The use according to claim 8 or 20, wherein menopause is drug induced, surgically induced, or naturally-occurring.

10. The use according to claim 9, wherein the drug is an anti-estrogen compound.

11. The use according to claim 10, wherein the anti-estrogen compound is tamoxifen.

12. The use according to claim 1, wherein the patient is a male patient

13. The use according to claim 12, wherein the male patient experiences drug induced hot flashes.

14. The use according to claim 13, wherein the drug is an anti-androgen compound.

15. The use according to claim 14, wherein the anti-androgen compound is leuprolide acetate.

16. The use according to claim 1 or 19, wherein said medicament is suitable for administration orally, parenterally, subcutaneously, intravenously, intramuscularly, intraperitoneally, by intranasal instillation, by implantation, by intracavitary or intravesical instillation, intraocularly, intraarterially, intralesionally, transdennally, or , by application to mucous membranes.

17. The use according to claim 1 or 19, wherein the medicament further comprises a pharmaceutically-acceptable carrier.

18. The use according to claim 17, wherein the medicament is in a liquid or solid dosage form.

19. Use of a tachykinin receptor antagonist in the preparation of a medicament for treating a symptom of honnonal variation in a postmenopausal female patient.

20. The use according to Claim 7 wherein the female patient is menopausal.

## Patentansprüche

1. Verwendung eines Tachykinin-Rezeptor-Antagonisten bei der Herstellung eines Medikamentes zur Behandlung von Hitzewallungen bei einem Patienten.

2. Verwendung nach Anspruch 1 oder 19, wobei der Tachykinin-Rezeptor-Antagonist ein NK₁-Rezeptor-Antagonist, ein NK₂-Rezeptor-Antagonist oder ein NK₃-Rezeptor-Antagonist oder eine Kombination hiervon ist.

3. Verwendung nach Anspruch 2, bei der der NK₁-Rezeptor-Antagonist aus der Gruppe ausgewählt ist, die GR 203040, CP 99994, CP122721, GR 205171, PD 154075, FK 888, RP 67580, L 760735, TAK 637, R 116301, Dapitant, L 754030, L 758298, MK 869, SR 140333, NKP 608, GR 73632, MEN 11467 und Kombinationen hiervon umfasst.

4. Verwendung nach Anspruch 2, wobei der NK₂-Rezeptor-Antagonist aus der Gruppe ausgewählt ist, die Saredutant, MEN 10627 und Kombinationen hiervon umfasst.

5. Verwendung nach Anspruch 2, wobei der NK₃-Rezeptor-Antagonist aus der Gruppe ausgewählt ist, die aus Talnetant-Hydrochlorid, Osanetant und Kombinationen hiervon besteht.

6. Verwendung nach Anspruch 1, wobei das Medikament den Tachykinin-Rezeptor-Antagonisten in einer Menge von ungefähr 10 mg bis ungefähr 5.000 mg pro täglicher Dosis umfasst.

7. Verwendung nach Anspruch 1, wobei der Patient eine Frau ist.

8. Verwendung nach Anspruch 7, wobei die Frau post-menopausal ist.

9. Verwendung nach Anspruch 8 oder 20, wobei die Menopause durch ein Medikament oder einen chirurgischen Eingriff induziert ist oder natürlicher Weise auftritt.

10. Verwendung nach Anspruch 9, wobei das Medikament eine Anti-Östrogen-Verbindung ist.

11. Verwendung nach Anspruch 10, wobei die Anti-Östrogen-Verbindung Tamoxifen ist.

12. Verwendung nach Anspruch 1, wobei der Patient ein Mann ist.

13. Verwendung nach Anspruch 12, wobei der männliche Patient unter Hitzewallungen leidet, die durch Medikamente induziert sind.

14. Verwendung nach Anspruch 13, wobei das Medikament eine Anti-Androgen-Verbindung ist.

15. Verwendung nach Anspruch 14, wobei die Anti-Androgen-Verbindung Leuprolid-Acetat ist.

16. Verwendung nach Anspruch 1 oder 19, wobei das Medikament für folgende Verabreichungsformen geeignet ist: oral, parenteral, subkutan, intravenös, intramuskulär, intraperitoneal, Einflößen in die Nase, Implantation, intracavitares oder intravesicales Eintröpfeln, intraokular, intraarteriell, intraläsional, transdermal oder durch Aufbringen auf Schleimhautmembranen.

17. Verwendung nach Anspruch 1 oder 19, bei der das Medikament weiterhin einen pharmazeutisch akzeptablen Träger umfasst.

18. Verwendung nach Anspruch 17, bei der das Medikament in einer flüssigen oder festen Dosierungsform vorliegt.

19. Verwendung eines Tachykinin-Rezeptor-Antagonisten bei der Zubereitung eines Medikamentes zur Behandlung eines Symptoms von hormonellen Veränderungen bei einer post-menopausalen Patientin.

20. Verwendung nach Anspruch 7, bei der die Patientin post-menopausal ist.

## Revendications

1. Utilisation d'un antagoniste de récepteur tachykinine dans la préparation d'un médicament pour traiter des bouffées de chaleur chez un patient.

2. Utilisation selon la revendication 1 ou 19, dans laquelle l'antagoniste de récepteur tachykinine est un antagoniste de récepteur NK₁, un antagoniste de récepteur NK₂ ou un antagoniste de récepteur NK₃, ou une de leurs combinaisons.

3. Utilisation selon la revendication 2, dans laquelle l'antagoniste de récepteur NK₁ est choisi dans le groupe constitué par GR 203040, CP 99994, CP 122721, GR 205171, PD 154075, FK 888, RP 67580, L 760735, TAK 637, R 116301, le dapitant, L 754030, L 758298, MK 869, SR 140333, NKP 608, GR 73632, MEN 11467, et leurs combinaisons.

4. Utilisation selon la revendication 2, dans laquelle l'antagoniste de récepteur NK₂ est choisi dans le groupe constitué par le sarédutant, MEN 10627, et leurs combinaisons.

5. Utilisation selon la revendication 2, dans laquelle l'antagoniste de récepteur NK₃ est choisi dans le groupe constitué par le chlorhydrate de talnétant, l'osanétant, et leurs combinaisons.

6. Utilisation selon la revendication 1, dans laquelle le médicament comprend l'antagoniste de récepteur tachykinine en une quantité d'environ 10 à environ 5000 mg par dose journalière.

7. Utilisation selon la revendication 1, dans laquelle le patient est une patiente.

8. Utilisation selon la revendication 7, dans laquelle la patiente est post-ménopausée.

9. Utilisation selon la revendication 8 ou 20, dans laquelle la ménopause est induite par médicament, par acte chirurgical, ou naturelle.

10. Utilisation selon la revendication 9, dans laquelle le médicament est un composé anti-oestrogène.

11. Utilisation selon la revendication 10, dans laquelle le composé anti-oestrogène est le tamoxifène.

12. Utilisation selon la revendication 1, dans laquelle le patient est un patient mâle.

13. Utilisation selon la revendication 12, dans laquelle le patient mâle souffre de bouffées de chaleur induites par médicament.

14. Utilisation selon la revendication 13, dans laquelle le médicament est un composé anti-androgène.

15. Utilisation- selon la revendication 14, dans laquelle le composé anti-androgène est l'acétate de leuprolide.

16. Utilisation selon la revendication 1 ou 19, dans laquelle ledit médicament convient pour une administration par voie orale, parentérale, sous-cutanée, intraveineuse, intramusculaire, intrapéritonéale, par instillation intranasale, par implantation, par , instillation intracavitaire ou intravésicale, par voie intraoculaire, intra-artérielle, intra-lésionnelle, transdermique, ou par application aux membranes muqueuses.

17. Utilisation selon la revendication 1 ou 19, dans laquelle le médicament comprend en outre un véhicule pharmaceutiquement acceptable.

18. Utilisation selon la revendication 17, dans laquelle le médicament est sous une forme posologique liquide ou solide.

19. Utilisation d'un antagoniste de récepteur tachykinine dans la préparation d'un médicament pour traiter un symptôme de variation hormonale chez une patiente post-ménopausée.

20. Utilisation selon la revendication 7, dans laquelle la patiente est ménopausée.
